# EUROPÄISCHE PATENTANMELDUNG

(11) **EP 2 011 779 A1**
(43) Veröffentlichungstag der Anmeldung: **07.01.2009**
(21) Anmeldenummer: 07111886.3
(22) Anmeldetag: 06.07.2007
(51) Int. Cl.: C04B 41/91, A61K 6/06, A61L 27/10

(54) **Keramischer Körper und Verfahren zu seiner Herstellung**

(71) Anmelder: Vita Zahnfabrik H. Rauter GmbH & Co. KG, 79713 Bad Säckingen (DE)
(72) Erfinder: Stephan, Marc, 79539 Lörrach (DE); Schöne, André, 79713 Bad Säckingen (DE)
(74) Vertreter: Meyers, Hans-Wilhelm

(57) **Zusammenfassung**

Verfahren zur Herstellung von Oberflächen keramischer Körper, wobei die folgenden Verfahrensschritte durchgeführt werden:
- Herstellung eines keramischen Rohlings
- Bearbeitung mindestens eines Teilbereiches der Oberfläche des keramischen Rohlings mit einem mechanischen Verfahren,
- gefolgt von einer chemischen Behandlung des mindestens einen Teilbereiches der mit dem mechanischen Verfahren behandelten Oberfläche des keramischen Rohlings,
- gefolgt von einer thermischen Behandlung des Rohlings, dessen Oberfläche der mechanischen und chemischen Behandlung unterzogen worden war,

Auch ein keramischer Körper erhältlich gemäß dem erfindungsgemäßen Verfahren wird beschrieben.

## Beschreibung

Gegenstand der vorliegenden Erfindung ist ein keramischer Körper, ein Verfahren zu seiner Herstellung und Verwendungen des keramischen Körpers insbesondere in der Medizin.

Technische Keramik oder Hochleistungskeramik verfügt über eine Reihe von Eigenschaften, die für zahlreiche Anwendungen im Maschinen- und Apparatebau, in der Verfahrens- und Fertigungstechnik, bei Hochtemperaturanwendungen, in der Präzisionstechnik, der Elektrotechnik und Elektronik sowie in der Optik Produkt verbessernd genutzt werden können. Manche Prozesse werden durch den Einsatz von Hochleistungskeramik überhaupt erst ermöglicht. Zu diesen Eigenschaften zählen:

Hochtemperatur- und Thermoschockbeständigkeit, hohe Festigkeiten und Zuverlässigkeit, auch im Bereich hoher Temperaturen, niedriger Wärmeausdehnungskoeffizient, Härte und Abriebfestigkeit, Beständigkeit gegen chemische Korrosion, niedriges spezifisches Gewicht höchste Steifigkeit, Langzeitfestigkeit (keine Ermüdung).

Sachkundig eingesetzt, lassen sich für den Anwender wirtschaftliche Vorteile gegenüber traditionellen Werkstoffen (Metalle, Kunststoffe, Glas und konventionelle Keramik) erzielen, beispielsweise durch eine verlängerte Lebensdauer von Bauteilen und Aggregaten oder durch Verbesserung des Wirkungsgrades von Reaktionen, infolge der Anwendung höherer Temperaturen. Einschränkungen des Einsatzes keramischer Werkstoffe, aufgrund z. B. deren Sprödigkeit, sind durch eine Konstruktion, die die speziellen Anforderungen der Keramik berücksichtigt vermeidbar.

Keramische Körper werden daher in der Technik auf sehr vielen Gebieten eingesetzt. Als Anwendungsgebiete sind neben technischen Gebieten insbesondere Medizin und Zahnmedizin zu nennen in der Keramiken bei Implantaten Verwendung finden. Dabei stehen sie in Konkurrenz zu Metallen wie Titan. Bisher können keramische Implantate aber nicht mit Oberflächentopographien ähnlich denjenigen von Titanimplantaten versehen werden, was sich negativ auf die Einheilung der Implantate auswirken kann.

Bisherige keramische Implantate besitzten maschinierte oder sandgestrahlte Oberflächen. Insbesonder die sandgestrahlte Oberfläche besitzt durch die scharfkantige Oberflächenschädigung und die oberflächlichen Strahlmittelrückstände keine optimalen mechanischen und mikrobiologischen Eigenschaften.

Ein technisches Problem der vorliegenden Erfindung besteht in der Schaffung einer keramischen Oberfläche, die die Nachteile des Standes der Technik vermeidet. Die keramische Oberfläche soll insbesondere das Anwachsen von Knochengewebe ermöglichen und bei Verwendung in medizinischen Implantaten deren Einheilung verbessern. Insbesondere soll eine Verkürzung der Einheilzeiten von keramischen Implantaten erreicht werden, so dass diese früher voll belastbar werden. Ein weiteres technisches Problem besteht in der Schaffung eines Verfahrens zur Herstellung von keramischen Oberfläche.

Gelöst wird das technische Problem durch ein Verfahren zur Herstellung von Oberflächen keramischer Körper, wobei die folgenden Verfahrensschritte durchgeführt werden:
- Herstellung eines keramischen Rohlings,
- Bearbeitung mindestens eines Teilbereiches der mit dem mechanischen Verfahren behandelten Oberfläche des keramischen Rohlings mit einem mechanischen Verfahren,
- gefolgt von einer chemischen Behandlung der Oberfläche des keramischen Rohlings,
- gefolgt von einer thermischen Behandlung des Rohlings, dessen Oberfläche der mechanischen und chemischen Behandlung unterzogen worden war.

Der im erfindungsgemäßen Verfahren einsetzbare Rohling kann, muss aber keine definierte Form aufweisen, wie die Form eines Dentalimplantats oder ähnliches.

Die Erfindung ermöglicht eine materialgerechte Erzeugung einer nanostrukturierten Mikrotopographie durch die Kombination der Verfahrensteilschritte. Die chemische Behandlung eröffnet Freiheitsgrade, die zur Justierung der Oberflächentopographie und -eigenschaften ausgenutzt werden kann. Die thermische Nachbehandlung bewirkt einen zusätzlichen Ätzeffekt, die sog. thermische Ätzung, wodurch zur Einstellung von gewünschten Eigenschaften zugemischten Oxide in kristallographisch/energetisch günstige Positionen der Kristallgitter diffundieren können und eine nanostrukturierte Oberfläche ohne "scharfe" Kanten hinterlassen. Dies gilt beispielsweise für Zirkoniumoxide, wobei durch Einstellung der chemischen Ätzzeit eine Mikrotopographie ohne die Bildung der unerwünschten Zirkoniumfluoride erzielt werden kann. Ein Vorteil besteht in der mit dieser Oberfläche zu erwartende schnellere biologische Integration eines Implantats in den Knochen.

Die Herstellung des keramischen Rohlings erfolgt beispielsweise durch formgebende Verfahren wie isostatischen Pressen, unter Erhalt eines Vorrohlings, der durch Sintern, insbesondere atmosphärisches Sintern und/oder heiß-isostatisches Pressen verdichtet wird. Neben einem atmosphärischen Sintern kommt auch ein Sintern unter einer Gasatmosphäre anderer Art in Betracht, um andere Eigenschaften zu verleihen, z. B. in Gegenwart von Wasserstoff. Weiterhin kommen als Verfahren zur Herstellung des Vorrohlings uniaxiale Pressen, keramisches Spritzgießen und Niederdruckspritzguss in Betracht. Dabei werden die zur Herstellung eines keramischen Rohlings üblicherweise eingesetzten oxidischen Bestandteile in pulverisierter Form den formgebenden Verfahren unterworfen. Die einschlägigen Verfahren sind beispielsweise für Trockenpressen und isostatisches Pressen in E. Kruse et al.: Technologie der Keramik-I Band 2: Mechanische Prozesse (1982) und bezüglich des heissisostatischen Pressens in J. Kriegesmann: Keramische Werkstoffe, Kapitel 3.6.3.0; B. W. Hofer, Heißisostatisches Pressen (1993) beschrieben. Der Vorrohling kann dann oberflächenbehandelt werden, wenn die Festigkeit ausreicht, das mechanische Verfahren der Oberflächenbehandlung zu überstehen.

Die oxidischen Bestandteile sind insbesondere Oxide der Metalle Aluminium, Zirkonium, Yttrium, Cer, Hafnium, Magnesium und in geringen Anteilen auch Eisen, Lanthan, Chrom, Strontium, Silizium, Kalzium.

Die Größe der im erfindungsgemäßen Verfahren einsetzbaren Partikel ist nicht kritisch, da alle in der Keramikherstellung üblichen Größen zum Einsatz gelangen können. Typischerweise bewegt sich die Partikelgröße in Bereichen von 0,1 µm bis 3 µm, insbesondere 0,3 µm bis 1 µm.

In einer Ausführungsform des erfindungsgemäßen Verfahrens wird der keramische Rohling in diesem Verfahrensschritt auf eine Dichte ≥ 90 % bis 99,9 % der theoretischen Dichte (100 % porenfrei) verdichtet.

Wenn die nach dem Verfahrensschritt der Herstellung des Vorrohlings sich ergebende Färbung unerwünscht ist, kann sich ein sogenannter Weißbrand empfehlen. Unter Weißbrand wird das Sintern oder das Tempern von Keramiken in oxidativer Atmosphäre nach dem heissisostatischen Pressen verstanden. Der hergestellte keramische Rohling kann zwecks weiterer Formgebung mittels materialabtragenden Verfahren bearbeitet werden. Als materialabtragendes Verfahren kommt zum Beispiel Schleifen, Fräsen, Laserabtragunsverfahren und/oder Polieren in Betracht.

Der wie oben beschrieben hergestellte keramisch Rohling, der gegebenenfalls mittels materialabtragender Verfahren in eine gewünschte Form gebracht wurde, wird in einem weiteren Schritt des erfindungsgemäßen Verfahrens in einem mechanischen Verfahrensschritt weiterbearbeitet. Der mechanische Verfahrensschritt zur Bearbeitung der Oberfläche des keramischen Rohlings bewirkt letztlich eine höhere Rauhigkeit der Oberfläche des keramischen Rohlings. Dabei ist es ausreichend, den Teil des Rohlings, welcher im Knochen verankert werden soll und gegebenenfalls den Implantathals aufzurauen.

Als mechanischer Verfahrensschritt kommt erfindungsgemäß beispielsweise ein Materialabstrahlungsverfahren wie ein Schleifverfahren, Fräsen, und/oder Laserabtragunsverfahren in Betracht. Das erfindungsgemäße Verfahren kann insbesondere durch Materialabstrahlungsverfahren mittels harter Partikel wie Korund-, Diamant, Siliziumkarbid durchgeführt wird. Dabei kann die Partikelgröße der harten Partikel 1 µm bis 250 µm betragen.

Bewährt hat sich insbesondere ein Sandstrahlverfahren. Beim Sandstrahlen kann mit einem Strahldruck von 1,5 bar bis 8 bar, insbesondere zwischen 4 bar und 6 bar gearbeitet werden. Als Strahlgut wird insbesondere Al₂O₃ verwendet. Die Korngröße des Strahlgutes, insbesondere Al₂O₃, beträgt 30 µm bis 250 µm, insbesondere 30 µm bis 130 µm.

An die mechanische Bearbeitung schließt sich erfindungsgemäß eine chemische Behandlung an. Erfindungsgemäß erfolgt die chemische Behandlung beispielsweise mittels Ätzung der Oberfläche des keramischen Rohlings durch Behandlung mit Flusssäure, flusssäurehaltigen Lösungen, Salpetersäure, Schwefelsäure und/oder Salzschmelzen. Typischerweise sind die flusssäurehaltigen Lösungen HF in Wasser. US-A-6,969,688 beschreibt Lösungsmittel sowie eine halogenhaltige Säure, wobei das Lösungsmittel mindesten eine der folgenden Komponenten beinhaltet: H₂O, Alkohol, Tetrahydrofuran (THF), Schwefelsäure (H₂SO₄) und Dimethylsulfoxid (DMSO), sowie die halogenhaltige Säure, die mindestens eine der folgenden Komponenten beinhaltet: HF, HBr, HI und HClO₄.

Als Ätzungsmittel kommen im erfindungsgemäßen Verfahren als Salzschmelzen, beispielsweise die in WO-A-2006/131010 genannten, in Betracht. Gemäß einer ersten bevorzugten Ausführungsform des Verfahrens handelt es sich bei der Salzschmelze um eine Salzschmelze aus Alkali-und/oder Erdalkali-Nitraten, Alkali- und/oder Erdalkali-Hydroxiden oder Alkali-und/oder Erdalkali-Halogenen, oder einer Mischung dieser Salze. Die Salzschmelze kann wenigstens ein Hydroxid, insbesondere ein Alkali-und/oder Erdalkali-Hydroxid, aufweisen. Einsetzbar sind als (eutektische) Salzschmelze solche, die ausschließlich bestehen aus einem oder mehreren Hydroxiden, insbesondere aus einem oder mehreren Alkali- und/oder Erdalkali-Hydroxiden. Die Mischungen können binär, ternär oder auch höher sein. Insbesondere wird eine Salzschmelze im wesentlichen aus Alkalihydroxiden, wie z.B. aus Kaliumhydroxid und/oder Natriumhydroxid und/oder Lithiumhydroxid verwendet. Geringe Bestandteile, typischerweise im Bereich von weniger als 5% oder sogar weniger als 2%, anderer Salze oder anderer Additive, sei es zur Einstellung der Ätzwirkung oder zur Einstellung der Schmelzetemperatur, können zusätzlich vorhanden sein.

Einsetzbar sind beispielsweise binäre Salzschmelzen z. B. aus Kaliumhydroxid und Natriumhydroxid, wobei diese beiden Komponenten in einem Verhältnis von 2 : 1 bis 0.5 : 1, insbesondere im Bereich von 1.5 :1 - 0.75 : 1 vorgelegt werden. Bei solchen binären Salzschmelzen insbesondere aus den genannten Komponenten wird beispielsweise bei einer Temperatur im Bereich von 100-600 °C, insbesondere bei einer Temperatur im Bereich von 150-250 °C gearbeitet.

Als geeignet erweisen sich auch beispielsweise ternäre Salzschmelzen aus Kaliumhydroxid, Natriumhydroxid und Lithiumhydroxid, wobei diese drei Komponenten in einem Verhältnis im Bereich von 10-20 : 4-10 : 0.5-2, insbesondere im Bereich von 14 : 6 : 1 vorgelegt werden. Bei solchen ternären Salzschmelzen kann bei einer Temperatur von 100 - 400°C gearbeitet werden, insbesondere bei einer Temperatur im Bereich von 150-250 °C.

Generell kann gesagt werden, dass typischerweise eine Salzschmelze bei einer Temperatur im Bereich von 80°C - 1300 °C verwendet werden kann, insbesondere im Bereich von 150°C - 600°C.

Die zu bearbeitende Oberfläche wird wenigstens bereichsweise über eine Dauer von 10 Minuten bis 300 Stunden oder von 10 bis 100 Stunden, insbesondere von 25 bis 35 Stunden einer Salzschmelze, z. B. in Form eines Bades, ausgesetzt. Es können aber auch andere Dauern Behandlungsdauer von wenigstens einer Stunde, weiter bevorzugt von je nach zu erzielenden Ergebnissen eingehalten werden.

Mit Hilfe der Dauer der Exposition des Rohling mit dem ätzenden Milieu lässt sich beispielsweise die Beschaffenheit der Oberfläche einstellen. So wird bei kurzzeitiger Einwirkung fluorhaltiger Säuren wie Flusssäure die Bildung von nennenswerten Mengen an Zirkoniumfluoriden unterdrückt. Gewünschtenfalls können Teilbereiche von der Ätzung ausgenommen werden, z.B. durch Vermeidung des Kontakts mit dem Ätzmittels oder Abdeckung der Oberfläche der Teilbereiche mit Substanzen, die einem Kontakt mit dem Ätzmittel standhalten, z.B. Wachs, PE, PP.

Das erfindungsgemäße Verfahren sieht im Anschluss an die mechanische und chemische Behandlung der Oberfläche des keramischen Rohlings einer thermischen Behandlung vor. Die thermische Behandlung wird typischerweise bei Temperaturen von 900 °C bis 1500 °C, insbesondere 1200 °C, 1400 °C, durchgeführt.

Die Verfahrensführung erfolgt erfindungsgemäß beispielsweise unter oxidativer Atmosphäre. Die Haltezeiten bei der gewünschten Endtemperatur betragen insbesondere 1 h bis 5 h.

Die thermische Behandlung erfolgt nach der mechanischen und chemischen Behandlung erfolgen, wobei die Schritte des erfindungsgemäßen Verfahrens können an Herstellungsverfahren des keramischen Rohlings nach dem Stand der Technik, angehängt werden können. Die mechanische Bearbeitung kann vor dem heißisostatischen Pressen erfolgen, gefolgt von der mechanischen Bearbeitung und der chemischen Behandlung gemäß des erfindungsgemäßen Verfahrens. Die thermische Behandlung kann kann auch mit dem heißisostatischen Pressen zusammenfallen. Auch kann der Prozess der Rohlingsherstellung und Oberflächenmodifikation zwischen dem Heiß-Isostatisch-Pressen und dem Weißbrand erfolgen. Der Weißbrand käme so dem abschließenden thermischen Prozess gleich.

Gegenstand der Erfindung ist auch ein keramischer Körper mit einer durch das erfindungsgemäße Verfahren erhältlichen Oberfläche. Der erfindungsgemäße keramische Körper weist eine einzigartige Oberfläche auf, die als Überlagerung einer durch das mechanische Behandeln eingeführten mikrostrukturierten Oberfläche und einer nanostrukturierten Oberfläche aufgefasst werden kann.

Der erfindungsgemäße keramische Körper kann beispielsweise in einer als medizinisches Implantat ausgestalteten Form verwendet werden. Insbesondere kommen Vorrichtungen wie Dentalimplantate, Endoprothesen, Knochennägel, Knochenschrauben (Kortikalisschrauben) und Platten in Betracht. Die erfindungsgemäßen keramischen Körper können auch als aktive Oberfläche wie als Träger für Katalysatoren, Filter und Adsorptionsmaterial eingesetzt werden.

Die Erfindung wird im folgenden beispielhaft näher erläutert.

### Beispiel 1:

Eine Probe aus 3Y-TZP-A wurde bei 1350°C vorgesintert und anschließend die Oberfläche mit 130 µm Korundpulver, 6 bar Druck und einem Strahlabstand von 4 cm sandgestrahlt. Nach Reinigung der Probe wurde diese in 38-40%iger Flusssäure 8 Stunden geätzt. Abschließend wurde die Probe gewässert, getrocknet und bei 1500°C eine Stunde nachgesintert.

Mit einem Rautiefenmessgerät Hommel Tester T8000 und einer Tastnadel mit Diamantspitze (2µm Radius und 60° Öffnungswinkel) wurde die resultierende Rautiefe bestimmt (DIN EN ISO 4287). Die Tastgeschwindigkeit betrug 0,15 mm/s:
Rₐ = 2,11µm ; R_{z} = 15,56µm ; Rₜ = 19,43µm und Rₘₐₓ = 18,51µm

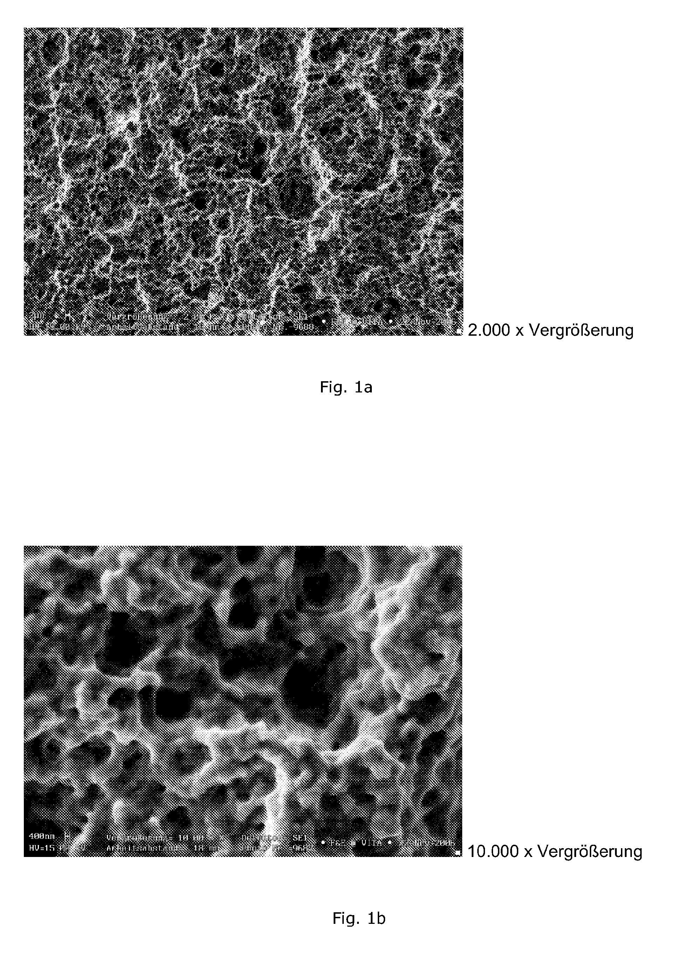

Fig. 1a und 1b zeigen die erhaltenen Oberflächen in 2 000 bzw 10 000 facher Vergrößerung

### Beispiel 2:

Eine Probe aus 3Y-TZP-A wurde bei 1350°C vorgesintert. Nach Reinigung der Probe wurde diese in 38-40%iger Flusssäure 8 Stunden geätzt. Abschließend wurde die Probe gewässert, getrocknet und bei 1500°C eine Stunde nachgesintert.

Mit einem Rautiefenmessgerät Hommel Tester T8000 und einer Tastnadel mit Diamantspitze (2µm Radius und 60° Öffnungswinkel) wurde die resultierende Rautiefe bestimmt (DIN EN ISO 4287). Die Tastgeschwindigkeit betrug 0,15 mm/s:
Rₐ = 1,86µm ; R_{z} = 15,82µm ; Rₜ = 18,64µm und Rₘₐₓ = 17,93µm

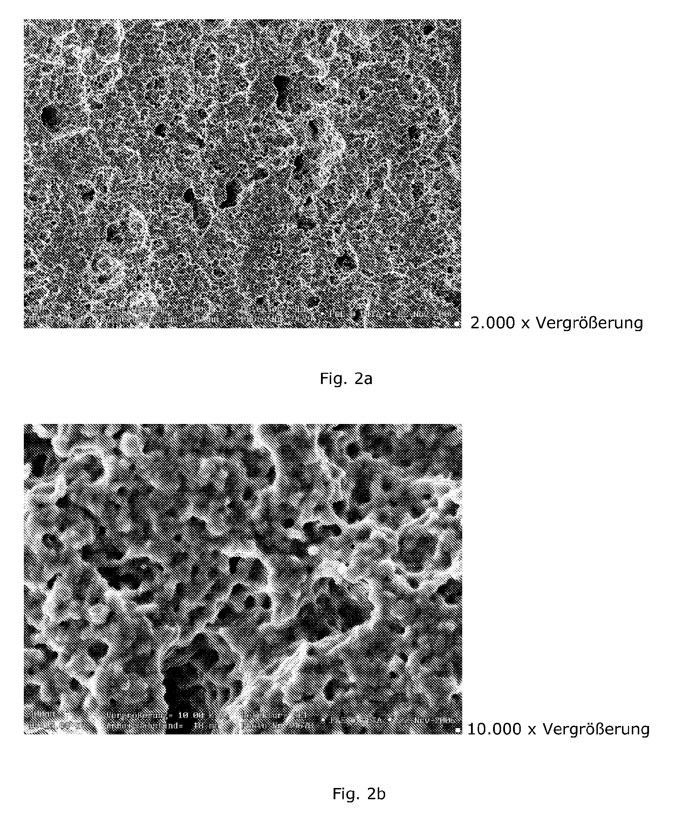

Fig. 2a und 2b zeigen die erhaltenen Oberflächen in 2 000 bzw 10 000 facher Vergrößerung.

### Beispiel 3

Eine Probe aus Y- TZP-A wurde bei 1500 °C vorgesintert und anschließend die Oberfläche mit 50 µm Korundpulver, 6 bar und einem Strahlabstand von 4 cm gesandstrahlt. Nach Reinigung der Probe wurde diese in 38-40%iger Flusssäure 1 Stunde geätzt. Abschließend wurde die Probe gewässert, getrocknet und bei 1400°C eine Stunde nachgesintert.

Mit einem Rautiefenmessgerät Hommel Tester T8000 und einer Tastnadel mit Diamantspitze (2µm Radius und 60° Öffnungswinkel) wurde die resultierende Rautiefe bestimmt (DIN EN ISO 4287). Die Tastgeschwindigkeit betrug 0,15 mm/s: Ra = 0,935 µm; R_{z} = 6,786 µm; Rₘₐₓ = 7,803 µm.

## Patentansprüche

1. Verfahren zur Herstellung von Oberflächen keramischer Körper, wobei die folgenden Verfahrensschritte durchgeführt werden:
- Herstellung eines keramischen Rohlings
- Bearbeitung mindestens eines Teilbereiches der Oberfläche des keramischen Rohlings mit einem mechanischen Verfahren,
- gefolgt von einer chemischen Behandlung des mindestens einen Teilbereiches der mit dem mechanischen Verfahren behandelten Oberfläche des keramischen Rohlings, gefolgt von einer thermischen Behandlung des Rohlings, dessen Oberfläche der mechanischen und chemischen Behandlung unterzogen worden war.

2. Verfahren nach Anspruch 1, wobei die Herstellung des keramischen Rohlings durch formgebende Verfahren wie isostatischen Pressen erfolgt, unter Erhalt eines Vorrohlings, der durch Sintern, insbesondere atmosphärisches Sintern oder Sintern in anderer Atmosphäre, und/oder heiß-isostatisches Pressen, keramisches Spritzgießen und Niederdruck-Spritzguß. verdichtet wird.

3. Verfahren nach mindestens einem der vorhergehenden Ansprüche, wobei der keramische Rohling auf eine Dichte ≥ 90 % bis 99,9 % der theoretischen Dichte verdichtet wird.

4. Verfahren nach Anspruch 2 und/oder 3, wobei ein Weißbrand nach der Herstellung des keramischen Rohlings erfolgt.

5. Verfahren nach mindestens einem der vorhergehenden Ansprüche, wobei der keramische Rohling zwecks Formgebung mittels materialabtragenden Verfahren bearbeitet wird.

6. Verfahren nach Anspruch 5, wobei das materialabtragende Verfahren Schleifen Fräsen, Laserabtragunsverfahren und/oder Polieren ist.

7. Verfahren nach mindestens einem der vorhergehenden Ansprüche, wobei das mechanische Verfahren zur Bearbeitung der Oberfläche des keramischen Rohlings eine höhere Rauhigkeit der Oberfläche des keramischen Rohlings bewirkt.

8. Verfahren nach Anspruch 7, wobei das mechanische Verfahren ein Materialabstrahlungsverfahren und/oder Schleifverfahren ist.

9. Verfahren nach Anspruch 7 und/oder 8, wobei das Materialabstrahlungsverfahren mittels harten Partikeln wie Korund-, Diamant, Silziumkarbid durchgeführt wird.

10. Verfahren nach mindestens einem der Ansprüche 7 bis 9, wobei die Partikelgröße der harten Partikel 1 µm bis 250 µm beträgt.

11. Verfahren nach mindestens einem der Ansprüche 7 bis 10, wobei das Materialabstrahlungsverfahren ein Sandstrahlen ist, mit einem Strahldruck von 1,5 bar bis 8 bar, insbesondere zwischen 4 bar und 6 bar durchgeführt wird, wobei das verwendete Strahlgut, insbesondere Al₂O₃, mit einer Korngröße von 30 µm bis 250 µm, insbesondere 30 µm bis 130 µm eingesetzt wird.

12. Verfahren nach mindestens einem der vorhergehenden Ansprüche, wobei die chemische Behandlung der Oberfläche des keramischen Rohlings vor und oder nach der mechanischen Bearbeitung der Oberfläche des keramischen Rohlings mittels Ätzung der Oberfläche des keramischen Rohlings erfolgt.

13. Verfahren nach Anspruch 12, wobei die Ätzung der Oberfläche des keramischen Rohlings durch Behandlung mit Flusssäure, flusssäurehaltigen Lösungen, Salpetersäure, Schwefelsäure, HBr, HI und HClO₄, ggf in Lösungsmitteln, und/oder Salzschmelzen erfolgt.

14. Verfahren nach Anspruch 13, wobei die flusssäurehaltigen Lösungen HF in Wasser ist.

15. Verfahren nach Anspruch 13, wobei die Salzschmelzen solche aus Alkali-und/oder Erdalkali-Nitraten, Alkali- und/oder Erdalkali-Hydroxiden oder Alkali- und/oder Erdalkali-Halogenen, oder einer Mischung dieser Salze sind.

16. Verfahren nach mindestens einem der vorhergehenden Ansprüche, wobei die thermische Behandlung im Anschluss an die mechanische und chemische Behandlung der Oberfläche des keramischen Rohlings bei Temperaturen von 900 °C bis 1500 °C durchgeführt wird.

17. Verfahren nach Anspruch 16, wobei die nachfolgende thermische Behandlung unter oxidativer Atmosphäre mit Haltezeiten bei Endtemperatur 1 h bis 5 h durchgeführt wird.

18. Keramischer Körper mit einer durch ein Verfahren nach einem der Ansprüche 1 bis 17 erhältlichen Oberfläche.

19. Keramischer Körper nach Anspruch 18, dessen Oberfläche eine Überlagerung einer durch das mechanische Behandeln eingeführten mikrostrukturierten Oberfläche und einer nanostrukturierten Oberfläche darstellt.

20. Verwendung des keramischen Körpers nach Anspruch 18 und/oder 19 in einer als medizinisches Implantat ausgestalteten Form.

21. Verwendung nach Anspruch 20 als Dentalimplantat, Endoprothese, Knochennägel, Knochenschrauben (Kortikalisschrauben) und Platten.

22. Verwendung des keramischen Körpers als aktive Oberfläche wie als Träger für Katalysatoren, Filter, Adsorptionsmaterial.
